# EUROPEAN PATENT APPLICATION

(11) **EP 2 524 964 A1**
(43) Date of publication of application: **21.11.2012**
(21) Application number: 11732843.5
(22) Date of filing: 07.01.2011
(51) Int. Cl.: C12N 15/09, C07K 16/28, G01N 33/574, C12P 21/08

(54) **ANTIBODY SPECIFIC TO ACTIVATED EGFR**

(30) Priority: 13.01.2010 JP 2010005115
(71) Applicant: Fujirebio Inc., Tokyo 103-0007 (JP)
(72) Inventor: ISE, Nobuyuki, Tokyo 103-0007 (JP); OMI, Kazuya, Tokyo 103-0007 (JP)
(74) Representative: Keirstead, Tanis Evelyne
(86) International application number: PCT/JP2011/050145
(87) International publication number: WO 2011/086973

(57) **Abstract**

Disclosed is a means for accurately measuring activated EGFR regardless of whether phosphorylation has occurred or not. The antibody or antigen-binding fragment thereof according to the present invention can bind to activated EGFR regardless of whether the activated EGFR is in a phosphorylated form or in a nonphosphorylated form. By carrying out an immunoassay using the antibody or antigen-binding fragment thereof, activated EGFR in a sample can be measured more accurately than by conventional methods. Abnormal activation of EGFR is responsible for the onset of cancer, and is regarded as a therapeutic target. Therefore, the antibody or antigen-binding fragment thereof according to the present invention is also useful for detection of cancers, in particular, cancers to which a molecular target drug(s) whose target is EGFR is(are) to be administered.

## Description

### TECHNICAL FIELD

The present invention relates to an antibody which specifically binds to activated EGFR, and a method of measuring activated EGFR and a method of detecting a cancer(s) utilizing the antibody.

### BACKGROUND ART

The epidermal growth factor receptor (EGFR) is a transmembrane receptor composed of an intracellular tyrosine kinase domain and autophosphorylation site; a transmembrane domain; and an extracellular EGF ligand-binding domain. Since abnormal activation of EGFR is known to be responsible for the onset of cancer, it has been regarded as a therapeutic target. Therefore, if a method that enables simple and accurate detection of activation of EGFR is provided, it may be very useful for elucidation of canceration mechanisms, development of therapeutic methods for cancer and diagnosis of cancer.

As a method for detecting activated EGFR kinase in a sample such as a cell or tissue extract, a method in which the phosphorylation state of EGFR, which varies with the activation, is measured as a marker for the activation (phosphorylated-EGFR-measurement method) is commonly employed. In general, phosphorylation of EGFR is interpreted synonymously with the activation. A method in which the activity to phosphorylate a synthetic peptide substrate is measured (enzyme-activity-measurement method) has also been proposed, although a general technique for the method has not been established yet because of a problem in sensitivity.

Further, antibodies that recognize sites of EGFR which show structural change are known (Non-patent Documents 1 and 2). The antibody described in Non-patent Document 1 recognizes the extracellular domain whose structure has been changed by undergoing high-mannose modification in the same domain. The antibody described in Non-patent Document 2 recognizes a site whose structure has been changed by phosphorylation.

### PRIOR ART DOCUMENTS

Non-patent Document 1: FASEB J 2005;19(7):780.
Non-patent Document 2: J Biol Chem 1995;270(14):7975-7979.

### DISCLOSURE OF THE INVENTION

### PROBLEMS TO BE SOLVED BY THE INVENTION

As explained above, the activation of EGFR is usually evaluated using the level of EGFR phosphorylation as an indicator. However, the present inventors focused attention to the point that the phosphorylation does not always reflect the activation of EGFR. That is, phosphorylation of EGFR occurs secondarily as a consequence of activation of EGFR, and does not always reflect activation of EGFR. In addition, EGFR may be cross-phosphorylated by other activated kinases in a biological sample even in cases where EGFR is not activated. Moreover, there are cases where the phosphorylation site selected to be measured is not phosphorylated although EGFR is activated. EGFR phosphorylation is affected by phosphatases in a living body, and measured values may largely vary depending on the conditions at the time of measurement. Phosphatases also strongly affect EGFR phosphorylation during preparation of a biological sample. Therefore, experiments must be carried out under low temperature using phosphatase inhibitors, and measured value may also vary if regulation by such means is insufficient. Thus, activation of EGFR cannot be appropriately measured by measurement methods based on the phosphorylation.

Of the above-described known antibodies which recognize structural changes of EGFR, the antibody described in Non-patent Document 2 is an antibody recognizing a site whose structure has been changed by phosphorylation, and therefore has the same problem as common techniques in which phosphorylation is used as an indicator. The structural change which the antibody described in Non-patent Document 1 recognizes does not always serve as an indicator of EGFR activation.

Accordingly, an object of the present invention is to provide means by which activated EGFR can be accurately measured regardless of whether phosphorylation has occurred or not.

### MEANS FOR SOLVING THE PROBLEMS

The present inventors intensively studied to produce antibodies using an EGFR intracellular domain as an immunogen and to succeed in obtaining antibodies which specifically recognize and bind to activated EGFR regardless of whether phosphorylation has occurred or not, thereby completing the present invention.

That is, the present invention provides an antibody or antigen-binding fragment thereof, which specifically binds to nondenatured activated EGFR. The present invention also provides a method of measuring activated EGFR, said method comprising: bringing a nondenatured sample into contact with the above-described antibody or antigen-binding fragment thereof according to the present invention; and measuring activated EGFR in the sample by immunoassay utilizing antigen-antibody reaction between the activated EGFR in the sample and said antibody or antigen-binding fragment thereof. The present invention further provides a method of detecting a cancer(s), comprising measuring activated EGFR in a sample separated from a living body by the above-described method according to the present invention. The present invention still further provides a kit for measurement of activated EGFR, said kit comprising the above-described antibody or antigen-binding fragment thereof according to the present invention. The present invention still further provides a kit for detection of a cancer(s), said kit comprising the above-described antibody or antigen-binding fragment thereof according to the present invention.

### EFFECTS OF THE INVENTION

By the present invention, an antibody by which activated EGFR *per se* can be directly measured without using the phosphorylation as an indicator was first provided. The antibody or antigen-binding fragment thereof according to the present invention specifically binds to nondenatured activated EGFR, regardless of whether the activated EGFR is in a phosphorylated form or in a nonphosphorylated form. Therefore, activated EGFR can be measured more accurately by the present invention than by conventional measurement methods. As well known in the art, abnormal activation of EGFR is responsible for the onset of cancer, and is regarded as a therapeutic target. The antibody or antigen-binding fragment thereof according to the present invention is also useful for detection of cancer, in particular, detection of cancer to which a molecular target drug(s) whose target is EGFR is(are) to be administered. The antibody or antigen-binding fragment thereof according to the present invention can also contribute to elucidation of the canceration mechanism and development of novel cancer therapy, and thus contribute greatly to diagnosis and treatment of cancer.

### BRIEF DESCRIPTION OF THE DRAWINGS

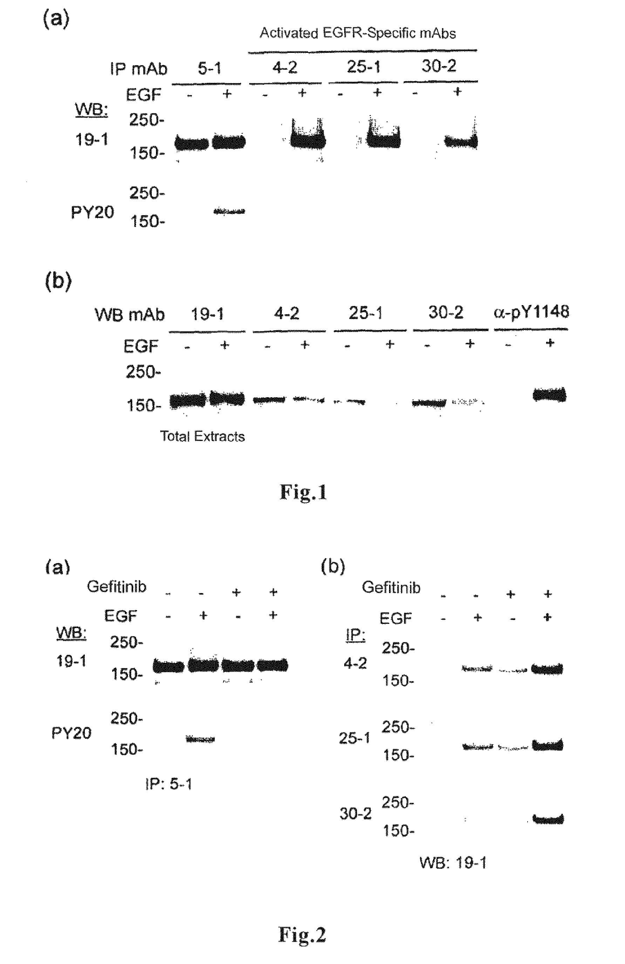

Fig. 1(a) shows the results of detection of EGFR in a cell extract immunoprecipitated with each of the antibodies prepared in Examples, which detection was carried out by Western blotting. At the top of the picture (IP mAb), the antibodies used for the immunoprecipitation are shown, and, in the left side of the picture (WB), the antibodies used for detection by Western blotting are shown. Fig. 1(b) shows the results of detection of EGFR in a denatured cell extract by Western blotting using each of the antibodies prepared in Examples.
Fig. 2 shows the results of EGFR immunoprecipitation experiments using each antibody, which experiments were carried out with a cell extract prepared by subjecting cells to ligand stimulation under the condition where phosphorylation was inhibited by gefitinib treatment. (a) shows the results obtained when the antibody 5-1 was used for the immunoprecipitation. In the left side (WB), the antibodies used for detection by Western blotting are shown. (b) shows the results obtained when each of the antibodies shown in the left side (IP) was used for the immunoprecipitation. For detection of the Western blot, the antibody 19-1 was used.

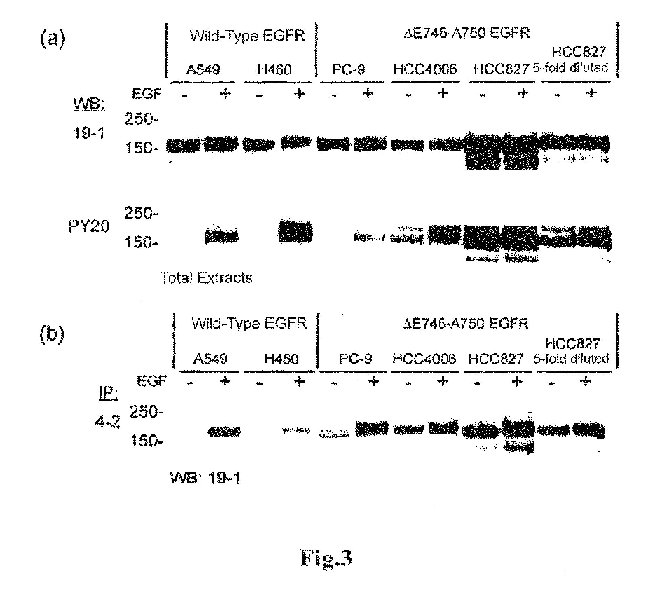

Fig. 3 shows the results of immunoprecipitation of EGFR in cell extracts using an activated EGFR-specific antibody, which immunoprecipitation was carried out on the cell extracts from human lung cancer-derived wild-type EGFR-expressing cell lines (A549 and H460), human lung cancer-derived activated EGFR mutant-expressing cell lines (PC-9 and HCC4006) and a human lung cancer-derived EGFR-overexpressing cell line (HCC827). (a) shows the results of detection of total extracts by Western blotting. In the left side (WB), the antibodies used for detection by Western blotting are shown. (b) shows the results of detection, by Western blotting, of EGFR which was immunoprecipitated with the antibody 4-2. For detection of the Western blot, the antibody 19-1 was used.

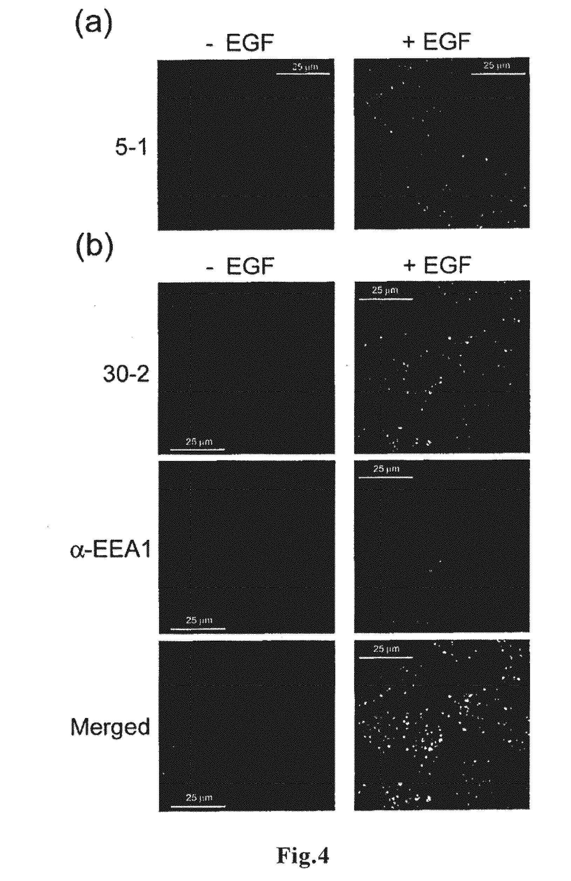

Fig. 4 shows the results of immunostaining of cells subjected to ligand stimulation, which immunostaining was carried out using an activated EGFR-specific antibody. (a) shows the results of immunostaining with the antibody 5-1. (b) shows the results of immunostaining with the antibody 30-2. The panel labeled as "Merged" shows a superimposed image of the image obtained by staining with the antibody 30-2 and the image obtained by staining with α-EEA1.

### MODE FOR CARRYING OUT THE INVENTION

The antibody according to the present invention specifically binds to activated EGFR which has not been denatured. That is, under conditions in which proteins have not been denatured, the antibody binds only to activated EGFR and does not substantially bind to EGFR which is not activated (nonactivated EGFR). The term "does not substantially bind" means that the antibody does not bind to nonactivated EGFR at a detectable level, or even if it binds to nonactivated EGFR at a detectable level, the degree of the binding is apparently weaker than that of the binding to activated EGFR so that it is clear for those skilled in the art that the antibody does not bind to activated EGFR.

"Activated EGFR" refers to EGFR whose structure has been changed so that it can transmit signals to downstream factors. EGFR is present in a nonactivated form in ordinary living cells. When EGF binds to the extracellular ligand-binding domain of EGFR, EGFR becomes activated by receiving this ligand stimulation, and then ATP binds to the ATP binding site in the intracellular domain, which leads to autophosphorylation and signal transduction to downstream factors. Known ligands which make EGFR activated include natural ligands such as TGF-α, amphiregulin, heparin-binding EGF-like growth factor (HB-EGF) and the like as well as EGF. In addition, activated mutants of EGFR which are in an activated state without ligand stimulation (e.g. deletion of a region of 746th Glu to 750th Ala in the EGFR sequence shown in SEQ ID NO: 2) are known, and human lung cancer-derived cell lines (e.g. PC-9, HCC4006) which express activated EGFR mutant are also known. EGFR overexpressing cell lines (e.g. HCC827) in which EGFR is activated by overexpression of EGFR so that downstream signaling pathway is running are also known. Activated EGFR recognized by the antibody according to the present invention includes various EGFR in an activated state such as those described above which those skilled in the art consider to be activated EGFR.

The antibody according to the present invention is an antibody which specifically binds to nondenatured (not being denatured) activated EGFR. The specificity of the present invention is lost under denatured conditions. That is, under the condition where original conformation of proteins is lost by denaturation of the proteins in a sample by physical treatment such as heat, freezing, ultra violet or the like or chemical treatment such as acid, base, guanidine, surfactant or the like, the antibody according to the present invention loses its specificity to activated EGFR and binds to not only activated EGFR but also nonactivated EGFR (see, Examples below), which suggests that the antibody according to the present invention recognizes conformational changes of EGFR associated with its activation.

The antibody according to the present invention can detect activated EGFR regardless of whether EGFR is phosphorylated or not. That is, the antibody can bind to both activated EGFR which has not been phosphorylated and activated EGFR which has been phosphorylated. As described above, activation of EGFR occurs prior to phosphorylation. However, in conventional measurement methods of activated EGFR, activation of EGFR is evaluated using phosphorylation as an indicator. By the antibody according to the present invention, activated EGFR *per se* can be directly detected, and therefore activation of EGFR can be evaluated more accurately than by conventional methods.

The base sequence and amino acid sequence of EGFR gene are known. For example, they have been registered in the NCBI database under accession Nos. NM_005228 (mRNA) and NP_005219 (protein). These sequences are shown in SEQ ID NOs: 1 and 2 in SEQUENCE LISTING. In the EGFR amino acid sequence, the N-terminal 24 residues (a region of aa1-24 in SEQ ID NO: 2) is the signal sequence, which 24 residues are removed after EGFR is expressed as a protein in a cell. The amino acid sequence shown in SEQ ID NO: 3 is the amino acid sequence of the EGFR protein in which the signal sequence is removed.

Examples of the antibody according to the present invention include, but not limited to, those which bind to a region within aa956-998 (which means "956th to 998th amino acids") or a region within aa1023-1115 of EGFR protein (SEQ ID NO: 3). In the following Examples, the three established antibodies were analyzed for binding property using various fragments of EGFR intracellular domain to confirm that the antibodies 4-2 and 25-1 bind to a region within aa956-998 and that the antibody 30-2 binds to a region within aa1023-1115. Thus, it is considered that the epitope of each antibody is present in each region.

Concerning activation and structural change of EGFR, there is a report about crystal structure analysis of EGFR kinase domain, which demonstrates that the structural change of the kinase domain is closely associated with the activation (Cancer Research 2004;64:6652-6659). The region of aa956-998 in which the epitopes of the antibodies 4-2 and 25-1 are present is consistent with the region demonstrated in the same report to be changed in structure by activation, which also suggests that the antibody according to the present invention recognizes the structural change by activation.

The antibody may be either a polyclonal antibody or monoclonal antibody, and a monoclonal antibody is preferred for immunoassays and the like because the reproducibility is high.

The anti-activated EGFR antibody according to the present invention may be prepared by, for example, the well-known hybridoma method using the intracellular domain of wild-type EGFR (the region of aa669-1210 in SEQ ID NO: 2; the region of aa645-1186 in SEQ ID NO: 3) as an immunogen. That is, the monoclonal antibody according to the present invention may be obtained by preparing an intracellular domain fragment of wild-type EGFR by the well-known genetic engineering technique; immunizing an animal (excluding human) with the prepared fragment; obtaining antibody-producing cells from the animal; fusing the obtained cells with immortalized cells such as myeloma cells to prepare hybridomas; screening a hybridoma which produces an antibody having the desired binding property; and collecting the antibody from the screened hybridoma.

Screening may be carried out from the following two viewpoints: (1) to confirm specific binding to the activated form using activated EGFR and nonactivated EGFR; and (2) to confirm binding to both nonphosphorylated activated EGFR and phosphorylated activated EGFR. Activated EGFR and nonactivated EGFR can be prepared by, for example, extracting proteins from known, wild-type EGFR-expressing cell lines (e.g. A549, H460) treated with EGF (e.g. addition of EGF to a culture medium) and untreated with EGF, respectively. Autophosphorylation of EGFR also occurs when cells are treated with the ligand in such a manner, and thus activated EGFR in a phosphorylated form is obtained. In order to obtain activated EGFR in a nonphosphorylated form, cells may be treated with the ligand in the presence of a phosphorylation inhibitor such as gefitinib or the like. Usually, the antibody according to the present invention can be appropriately obtained by screening antibodies prepared by a conventional method based on the specific binding to activated form; and then by screening the obtained antibodies to select an antibody which binds to activated EGFR regardless of whether EGFR is phosphorylated or not. The meaning of "specific binding" as used herein is the same as the definition described above.

Antigen-binding fragments can be prepared from the antibody according to the present invention. The term "antigen-binding fragment" means an antibody fragment which retains a binding property (antigen-antibody reactivity) to the corresponding antigen of the antibody such as Fab fragment or F(ab')₂ fragment of immunoglobulin. It is well-known that such antigen-binding fragments may be used in immunoassays, and these fragments are also useful similarly to the original antibody. As is well-known, Fab fragment and F(ab')₂ fragment can be obtained by treating a monoclonal antibody with a protease such as papain or pepsin. It should be understood that an antigen-binding fragment is not restricted to a Fab fragment or a F(ab')₂ fragment, and may be any fragment which retains a binding property to the corresponding antigen, including those prepared by a genetic engineering technique. For example, an antibody prepared by expressing a single chain fragment of variable region (scFv) in *E*. *coli* by a genetic engineering technique may also be used. A method of producing scFv is also well-known, and scFv can be prepared by extracting mRNA from hybridoma prepared in the above-described manner to prepare single chain cDNA; carrying out PCR using primers specific for immunoglobulin H chain and L chain to amplify the immunoglobulin H chain gene and L chain gene; ligating them with a linker; adding an appropriate restriction site(s) thereto; introducing it into a plasmid vector; transforming *E. coli* with the vector; and collecting scFv from *E*. *coli.* Such a scFv is included in the scope of the present invention as an "antigen-binding fragment".

Because an antibody and its antigen-binding fragment which can exhibit specificity to activated EGFR without being affected by whether EGFR is phosphorylated or not are provided by the present invention, an immunoassay using the antibody or antigen-binding fragment thereof makes it possible to measure activated EGFR in a sample more accurately than conventional methods. That is, the present invention also provides a method of measuring activated EGFR, which method comprises measuring activated EGFR in a sample by immunoassay utilizing antigen-antibody reaction between the activated EGFR in the sample and the above-described antibody or antigen-binding fragment thereof according to the present invention. It is noted here that, in the present invention, the term "measurement" includes "detection", "quantification" and "semi-quantification".

As described above, the specificity to activated EGFR that the antibody according to the present invention has is exhibited under the condition where activated EGFR has not been denatured. Therefore, when carrying out measurement of activated EGFR using the antibody or antigen-binding fragment thereof according to the present invention, it is necessary to bring proteins in a sample into contact with the anti-activated EGFR antibody or antigen-binding fragment thereof under the condition where the sample has not been subjected to protein denaturation.

Immunoassays *pe*r *se* are well-known in the art. Any of well-known immunoassays may be employed as long as the above-described requirement that a nondenatured sample should be brought into contact with the antibody or antigen-binding fragment thereof according to the present invention can be satisfied. That is, when classifying on the basis of the reaction type, known immunoassays include sandwich methods, competition methods, agglutination methods and the like, and when classifying on the basis of the label employed, known immunoassays include enzyme immunoassays, radio immunoassays, fluorescence immunoassays and the like. Any of these are included in the term "immunoassays" referred to in the present invention and may be employed in the measurement method according to the present invention. Specific examples of the immunoassay preferably employed include, but not limited to, immunoprecipitation, ELISA, immunostaining and the like.

Reagents necessary for each type of immunoassays are also well-known. The immunoassay can be carried out using an ordinary immunoassay kit except that the antibody or antigen-binding fragment thereof according to the present invention is used. That is, the present invention also provides a measuring kit for carrying out the measurement method according to the present invention, which kit comprises the above-described antibody or antigen-binding fragment thereof according to the present invention. Other reagents contained in the kit besides the antibody or antigen-binding fragment thereof may be the same as in known ordinary immunoassay kits.

Samples are not restricted at all, and any sample can be used as long as it may contain activated EGFR. The sample may be a living body-derived sample separated from a living body, or may be a sample not derived from a living body (e.g. an activated EGFR sample prepared by a genetic engineering technique in a laboratory). Examples of the living body-derived sample include, but not limited to, blood samples (whole blood, plasma, serum, etc.), cell extracts, tissue samples and the like. The living body is, but not limited to, a mammal such as human, dog, cat, rabbit, mouse, hamster or the like.

As is well-known in the art, abnormal activation of EGFR is responsible for the onset of cancer, and regarded as a therapeutic target. As concretely demonstrated in the following Examples, activated EGFR can be detected in various cancer-derived cell lines by using the antibody according to the present invention. It is known that more amount of activated EGFR is present in certain cancers such as lung cancer than in healthy individuals. Molecular target drugs whose target is EGFR such as gefitinib are effective against such types of cancers in which EGFR is abnormally activated. A cancer(s) in a living body can be detected by carrying out the method of measuring activated EGFR according to the present invention on a sample separated from the living body to be determined whether the body has a cancer(s). Especially, the method of measuring activated EGFR is useful for investigating whether a molecular target drug(s) whose target is EGFR should be administered to the cancer(s) or not. Therefore, the method of measuring activated EGFR according to the present invention is also useful as a method of detecting a cancer(s), in particular, as a method of detecting a cancer(s) to which a molecular target drug(s) whose target is EGFR is(are) to be administered. Similarly to the method, the above-described kit for measurement of activated EGFR is also useful as a kit for detecting a cancer(s), in particular, as a kit for detecting a cancer(s) to which a molecular target drug(s) whose target is EGFR is(are) to be administered.

When it is uncertain whether the living body to be subjected to the present invention has a cancer(s) or not, the measurement method according to the present invention may be carried out on a sample separated from the living body to measure activated EGFR in the sample, by which whether the living body has a cancer(s) can be judged. If the measured value is significantly higher than the amount of activated EGFR in samples derived from healthy individuals, there is a high possibility that the living body is suffering from cancer. In the case where such a living body has been definitely diagnosed with cancer, a molecular target drug whose target is EGFR may be useful for treatment of the cancer in the living body, and therefore the cancer may be effectively treated by actively using such a molecular target drug. That is, the detection method according to the present invention can also be applied to a living body with already diagnosed cancer, for the purpose of considering whether to use a molecular target drug(s). As a cancer with activated EGFR, lung cancer, colon cancer, breast cancer and the like are known. However, without limitation to these known examples, a molecular target drug whose target is EGFR may be effective against any cancers in which activation of EGFR is actually confirmed by the method according to the present invention, and therefore the scope of the present invention is not restricted by the kind of cancers.

The term "molecular target drug" is a common term in the field of medical therapy, and usually refers to a drug which specifically acts on the target gene or gene products associated with a disease. In the present invention, the term "molecular target drug whose target is EGFR" refers to a drug which acts on EGFR as a target, and includes those which bind to nonactivated EGFR and inhibit conversion into the active form or inhibit signaling to downstream factors, or those which bind to activated EGFR and inhibit signaling to downstream factors. Known molecular target drugs whose target is EGFR include gefitinib, erlotinib and the like, and various novel substances have been under development. By using the antibody or antigen-binding fragment thereof according to the present invention, activated EGFR can be measured more accurately than by conventional methods, and therefore cancer patients with excessively activated EGFR can be preferably detected, which makes a great contribution to the selection of therapeutic approach.

In the case of cancer detection, it is preferred, from the viewpoint of increasing the measurement accuracy, to determine the normal reference value by measuring the amount of activated EGFR in a plurality of healthy individuals, and then to compare the amount of activated EGFR in the subject sample with the normal reference value to examine whether there is a significant difference. It is more preferred to further determine the cancer reference value by measuring the amount of activated EGFR in a plurality of known cancer patients with excessively activated EGFR, and then to examine whether there is a significant difference between the amount of activated EGFR in the subject sample and the cancer reference value, by which the measurement accuracy can be further increased. Graded cancer reference values such as the false-positive reference value, the positive reference vale and the like may also be determined and used. It is not necessary to determine these reference values at each time when the present invention is carried out, and the predetermined reference values may be used.

In addition, the antibody or antigen-binding fragment thereof according to the present invention may be used in, for example, screening of novel compounds useful for cancer therapy or the like. The antibody or antigen-binding fragment thereof according to the present invention can contribute to cancer diagnosis and selection of therapeutic approach, as well as elucidation of the canceration mechanism and development of novel cancer therapy.

### EXAMPLES

The present invention will now be described more concretely by way of an example thereof. However, the present invention is not restricted to the example below.

### 1. Preparation of Antibody

Mice were immunized with an EGFR intracellular domain (the region corresponds to aa669-1210 in SEQ ID NO:2 and aa645-1186 in SEQ ID NO:3) prepared by expression in insect cells according to a conventional method, which immunization was carried out with Freund's adjuvant. Hybridomas producing antibodies against the EGFR intracellular domain were produced according to the conventional hybridoma method. Reactivity of the antibodies against the immunogen was analyzed by ELISA, and hybridomas producing antibodies that bound to the immunogen were selected, followed by recovering the antibodies (antibodies 4-2, 5-1, 19-1, 25-1 and 30-2). As shown in the experiments below, 5-1 and 19-1 were antibodies that bound to EGFR in any state (that is, nonspecific to activated EGFR), and 4-2, 25-1 and 30-2 were antibodies that specifically bound to activated EGFR.

### 2. Epitope Analysis

The fragments of the EGFR intracellular domain shown in Table 1 below were expressed in *E*. *coli* as GST fusion proteins, and these were subjected to SDS-PAGE and then transferred to a PVDF membrane. Western blotting was carried out with the established antibodies, and the region where all the reacted fragments were overlapping was determined as the epitope of each antibody. The reactivity of the respective established antibodies to the respective fragments is shown in Table 1. It was confirmed that the established antibodies 4-2 and 25-1 recognized the site (aa956-998) adjacent to the C-terminus of the kinase domain of EGFR, and the antibody 30-2 recognized the region of aa1023-1115. The region of aa956-998 corresponds to the region which has been shown, by crystal structure analysis, to undergo structural change (Cancer Research 2004; 64; 6652-6659).

**[Table 1]**

| Recombinant No. | Fragment (position in SEQ ID NO:3) | Reactivity | | |
|---|---|---|---|---|
| | | Antibody 4-2 | Antibody 25-1 | Antibody 30-2 |
| 1 | aa688-955 | - | - | - |
| 2 | aa770-998 | + | + | - |
| 3 | aa770-852 | - | - | - |
| 4 | aa831-998 | + | + | - |
| 5 | aa956-1115 | + | + | + |
| 6 | aa956-1022 | + | + | - |
| 7 | aa1023-1043 | - | - | - |
| 8 | aa1023-1186 | - | - | + |

### 3. Analysis of Specificity to Activated EGFR

Cell extracts of A549 with or without EGF treatment were subjected to immunoprecipitation of EGFR using each established antibody and the antibody 5-1, which recognized EGFR in any state. Immunoprecipitated EGFR was subjected to SDS-PAGE and then transferred to PVDF, and immunoprecipitated EGFR was detected by Western blotting with the anti-EGFR antibody 19-1, which recognized EGFR in any state (Fig. 1(a)). The established antibodies 4-2, 25-1 and 30-2 detected EGFR only in the EGF-treated cell extract, which confirmed that these antibodies specifically immunoprecipitated EGFR which was activated by EGF ligand stimulation.

### 4. Analysis of Phosphorylation Independence

Cell extracts of A549 with or without EGF treatment were denatured with an SDS sample buffer and subjected to SDS-PAGE. The resultant was then transferred to a PVDF membrane, and subjected to detection by Western blotting using the established antibodies (Fig. 1(b)). The antibody α-pY1148 in the figure is an antibody established by using as an immunogen a peptide synthesized based on the sequence surrounding pY1148, which is one of the phosphorylation domains of EGFR. As shown in Fig. 1(b), unlike the phosphorylated-EGFR-specific antibody α-pY1148 (right end), the established antibodies 4-2, 25-1 and 30-2 reacted with the denatured EGFR transferred to the PVDF membrane, irrespective of whether EGF stimulation was performed or not.

An immunoprecipitation experiment was carried out in the same manner as in the above-described 3 in the presence of an EGFR-specific inhibitor gefitinib (Fig. 2(b)). Even under the condition where phosphorylation was inhibited (gefitinib +), the established antibodies 4-2, 25-1 and 30-2 specifically immunoprecipitated EGFR which was activated by EGF ligand stimulation. By this, it was confirmed that the established antibodies 4-2, 25-1 and 30-2 can recognize non-denatured EGFR activated by EGF ligand stimulation regardless of whether EGFR is phosphorylated or not.

### 5. Analysis of Reactivity against EGFR Activated by Activation Mutation or Overexpression

Cell extracts of lung cancer-derived cell lines showing expression of wild-type EGFR, expression of activated EGFR mutant, or overexpression of EGFR were subjected to immunoprecipitation with the established antibodies. Immunoprecipitated EGFR was subjected to SDS-PAGE and then transferred to a PVDF membrane. The resultant was then subjected to detection by Western blotting using the EGFR antibody 19-1, which recognized EGFR in any state (Fig. 3). The established antibody 4-2 showed reactivity against EGFR in an EGF-stimulation-dependent manner in the cell lines expressing wild-type EGFR (A549 and H460); however, in the cases of the mutant EGFR-expressing cell lines (PC-9 and HCC4006) and the EGFR-overexpressing cell line (HCC827), the antibody reacted with EGFR even under the condition where EGF stimulation was not carried out. These mutant cell lines and overexpressing cell line are known to be cell lines derived from human cancers in which EGFR is activated, and it was therefore shown that the established antibodies can specifically react with various types of activated EGFR, including those expressed in such cancers. The activated EGFR mutants with which the established antibodies specifically reacted are the therapeutic target of molecular target drugs for cancer such as gefitinib, and it was therefore shown that the established antibodies are also useful for diagnosis of cancers, such as diagnosis of cancers to be treated with molecular target drugs.

### 6. Detection of Activated EGFR by Immunocytostaining System

Untreated and EGF-treated A549 cells were fixed with PFA, and subjected to permeabilization with a buffer containing Triton X-100. The anti-EGFR antibody 5-1, which recognizes EGFR in any state, was allowed to react with the cells, and the reacted antibody was detected with an anti-mouse Alexa-Fluor-488-fluorescence-labeled secondary antibody (Fig. 4(a)). Alternatively, the activated EGFR-specific antibody 30-2 and an anti-EEA1 rabbit antibody were reacted with the cells, and thereafter, the reacted antibody 30-2 was detected with an Alexa-Fluor-488-fluorescence-labeled secondary antibody, and the reacted anti-EEA1 rabbit antibody was detected with an Atexa-Fluor-555-fluorescence-labeled secondary antibody (Fig. 4(b)). As a result, it was shown that the antibody 5-1 reacted with EGFR localized on the cell membrane of the EGF-untreated A549 cells. In contrast, the antibody 30-2 did not react with EGFR of the EGF-untreated A549 cells, but reacted specifically with EGFR localized in early endosomes together with EEA1 in the A549 cells subjected to EGF stimulation.

## Claims

1. An antibody or antigen-binding fragment thereof, which specifically binds to nondenatured activated EGFR.

2. The antibody or antigen-binding fragment thereof according to claim 1, which binds to both phosphorylated activated EGFR and nonphosphorylated activated EGFR.

3. The antibody or antigen-binding fragment thereof according to claim 1 or 2, which substantially does not bind to nondenatured nonactivated EGFR.

4. The antibody or antigen-binding fragment thereof according to any one of claims I to 3, which binds to a region within aa956-998 or a region within aa1023-1115 of the amino acid sequence of EGFR protein shown in SEQ ID NO: 3.

5. The antibody or antigen-binding fragment thereof according to any one of claims 1 to 4, wherein said antibody is a monoclonal antibody.

6. A method of measuring activated EGFR, said method comprising: bringing a nondenatured sample into contact with the antibody or antigen-binding fragment thereof according to any one of claims 1 to 5; and measuring activated EGFR in the sample by immunoassay utilizing antigen-antibody reaction between the activated EGFR in the sample and said antibody or antigen-binding fragment thereof.

7. A method of detecting a cancer(s), comprising measuring activated EGFR in a sample separated from a living body by the method according to claim 6.

8. The method according to claim 7, which is a method of detecting a cancer(s) to which a molecular target drug(s) whose target is EGFR is(are) to be administered.

9. A kit for measurement of activated EGFR, said kit comprising the antibody or antigen-binding fragment thereof according to any one of claims 1 to 5.

10. A kit for detection of a cancer(s), said kit comprising the antibody or antigen-binding fragment thereof according to any one of claims 1 to 5.

11. The kit according to claim 10, which is a kit for detecting a cancer(s) to which a molecular target drug(s) whose target is EGFR is(are) to be administered.
